# EUROPÄISCHE PATENTSCHRIFT

(11) **EP 3 134 504 B1**
(45) Veröffentlichungstag und Bekanntmachung des Hinweises auf die Patenterteilung: **11.01.2023**
(21) Anmeldenummer: 15727881.3
(22) Anmeldetag: 09.06.2015
(51) Int. Cl.: C12M 1/02, C12M 3/06, C12M 1/00

(54) **EINLEGEBODEN UND INKUBATOR**
INSERT SHELF AND INCUBATOR
TABLETTE ET INCUBATEUR

(30) Priorität: 14.08.2014 DE 102014011941
(43) Veröffentlichungstag der Anmeldung: 01.03.2017
(73) Patentinhaber: IKA - Werke GmbH & Co. KG, 79219 Staufen (DE)
(72) Erfinder: FREI, Andreas, 79219 Staufen (DE)
(74) Vertreter: Rummler, Felix
(86) Internationale Anmeldenummer: PCT/EP2015/001154
(87) Internationale Veröffentlichungsnummer: WO 2016/023603

(56) Entgegenhaltungen:
- EP-A1- 2 180 037
- EP-A2- 2 489 435
- EP-A2- 2 722 385
- DE-A1-102006 021 852
- DE-A1-102013 000 044
- DE-B3-102008 010 780
- DE-T2-602004 006 508
- DE-T2-602004 011 334

## Beschreibung

Die Erfindung betrifft einen in einen Inkubator einsetzbaren und in Gebrauchsstellung in den Inkubator eingesetzten Einlegeboden zur Aufnahme wenigstens eines Inkubationsgefäßes.

Ferner betrifft die Erfindung auch einen Inkubator mit einem beheizbaren Inkubationsraum und mit wenigstens einem in Gebrauchsstellung in den Inkubationsraum des Inkubators eingesetzten und/oder eingeschobenen, auswechselbaren Einlegeboden zur Aufnahme wenigstens eines, ein Inkubationsmedium enthaltenden Inkubationsgefäßes.

Derartige Einlegeböden und derartige Inkubatoren sind aus der Praxis in verschiedenen Ausführungsformen bekannt.

So sind Einlegeböden für Inkubatoren bekannt, die, ähnlich wie Bleche in einen Backofen, in den Inkubator eingeschoben werden können. In ihrer eingeschobenen Gebrauchsstellung können auf die Einlegeböden dann Inkubationsgefäße aufgestellt werden, um die in den Inkubationsgefäßen befindlichen Inkubationsmedien im Inneren des Inkubators zu erwärmen, also einer Inkubationsbehandlung zu unterziehen.

EP 2 722 385 offenbart einen Klimaaufbewahrungsschrank für Zellkulturen. Der Inkubator verfügt über einen von außerhalb des Inkubators agierenden magnetischen Antrieb, um die Stapelregaltürme im Inkubator auf einer Kreisbahn zu einem Ausgabeplatz mittels Magnetantrieb zu bewegen Aus der Druckschrift DE 10 2006 021 852 A1 ist ein Inkubator mit einem einsetzbaren und/oder einschiebbaren Einlegeboden zur Aufnahme eines Inkubationsgefäßes bekannt, wobei der Einlegeboden mit einem Mittel versehen ist, mit dem ein Inkubationsmedium, welches sich in dem auf den Einlegeboden gestellten Inkubationsgefäß befindet, in Bewegung versetzbar ist. Aus der Druckschrift DE 10 2008 010 780 B3 ist ein Schüttelinkubator mit einem Schütteltisch bekannt, wobei der Schütteltisch fest mit einem Antriebsarm verbunden ist.

Je nach Anwendungsfall kann es dabei vorteilhaft sein, den Inhalt der Inkubationsgefäße während der Inkubationsbehandlung auch zu bewegen. Dazu ist es bekannt, sogenannte Schüttelinkubatoren zu verwenden, die beispielsweise als Schüttler mit Inkubator-Haube oder aber als auf einen Schütteltisch aufstellbare Inkubatoren ausgebildet sind.

Insbesondere bei auf einen Schütteltisch aufgestellten Inkubatoren kann dabei nachteilig sein, dass bei dieser Lösung der gesamte Inkubator bewegt werden muss, um das im Verhältnis zu dem Inkubator relativ kleine und leichte Inkubationsgefäß samt Inhalt in Bewegung zu versetzen. Um das Inkubationsmedium in gewünschter Art und Weise zu bewegen muss bei den vorbekannten Schüttelinkubatoren eine unnötig große Masse mitbewegt werden, was mit einem entsprechend hohen Energiebedarf verbunden sein kann.

Aufgabe der Erfindung ist es daher, einen eingangs definierten Einlegeboden und einen eingangs definierten Inkubator zu schaffen, die eine effizientere Bewegung von Inkubationsmedien während einer Inkubationsbehandlung erlauben.

Diese Aufgabe wird bei dem eingangs definierten Einlegeboden durch die Merkmale des Patentanspruchs 1 gelöst. Insbesondere wird diese Aufgabe dadurch gelöst, dass der eingangs definierte Einlegeboden mit wenigstens einem Mittel versehen ist, mit dem ein Inkubationsmedium, das sich in dem auf den Einlegeboden in Gebrauchsstellung abgestellten Inkubationsgefäß befindet, in Bewegung versetzbar ist.

Auf diese Weise kann das Inkubationsmedium in dem wenigstens einen Inkubationsgefäß mit Hilfe des Einlegebodens in Bewegung versetzt werden, ohne dass der gesamte Inkubator bewegt werden muss. Ferner ist es so möglich, bereits bestehende Inkubatoren oder auch als Wärmeschränke ausgebildete Inkubatoren mit einem derartigen Einlegeboden nachzurüsten und auf diese Weise den Funktionsumfang bestehender Inkubatoren zu erweitern.

Besonders zweckmäßig kann es sein, wenn das wenigstens eine Mittel elektrisch betreibbar ist. Alternativ oder zusätzlich dazu kann das wenigstens eine Mittel zudem mechanisch bewegbar sein und durch seine mechanische Bewegung das wenigstens eine auf den Einlegeboden in Gebrauchsstellung aufgestellte Inkubationsgefäß und ein darin befindliches Inkubationsmedium zumindest indirekt in die gewünschte Bewegung versetzen.

Bei einer bevorzugten Ausführungsform der Erfindung kann vorgesehen sein, dass das wenigstens eine Mittel des Einlegebodens in Bezug auf einen in Gebrauchsstellung feststehenden Rahmen oder Einschubrahmen des Einlegebodens mechanisch bewegbar ist. So ist es möglich, dass der Einlegeboden mittels seines feststehenden Rahmens oder Einschubrahmens in einen Inkubator eingeschoben werden und relativ zu dem Inkubator selbst feststehen kann, während das wenigstens eine Mittel relativ zu dem feststehenden Rahmen oder Einschubrahmen und damit relativ zu dem Inkubator mechanisch bewegt werden kann, um das Inkubationsmedium in dem wenigstens einen auf den Einlegeboden aufgestellten Inkubationsgefäß in die gewünschte Bewegung zu versetzen.

Erfindungsgemäß ist vorgesehen, dass der Einlegeboden als wenigstens ein Mittel zur Bewegung des Inkubationsmediums eine in zumindest einer Bewegungsrichtung bewegliche Aufstellfläche zur Aufnahme wenigstens eines Inkubationsgefäßes und einen gegenüber der bewegliche Aufstellfläche feststehenden Rahmen, beispielsweise den bereits zuvor erwähnten Einschubrahmen, aufweist, mit dem der Einlegeboden in seiner in den Inkubator eingesetzten Gebrauchsstellung festlegbar ist.

Gemäß der Erfindung weist der Einlegeboden eine Antriebsvorrichtung auf, mit der die bewegliche Aufstellfläche des Einlegebodens zumindest mittelbar bewegbar ist. Zusätzlich weist der Einlegeboden erfindungsgemäß eine Antriebsvorrichtung auf, mit der das in einem auf den Einlegeboden gestellten Inkubationsgefäß befindliche Inkubationsmedium zumindest mittelbar in Bewegung versetzbar ist.

Gemäß einer Variante der Erfindung ist vorgesehen, dass der Einlegeboden und/oder eine, beispielsweise die bereits zuvor erwähnte, Aufstellfläche des Einlegebodens, als ein Mittel zu Bewegung des Inkubationsmediums wenigstens einen Rührmagnetantrieb aufweist, mit dem ein umlaufendes Magnetfeld zum Antrieb wenigstens eines in ein Inkubationsgefäß einsetzbaren Rührmagneten erzeugbar ist.

Dieser wenigstens eine Rührmagnetantrieb kann mittels einer, beispielsweise der bereits erwähnten, Antriebsvorrichtung des Einlegebodens angetrieben werden.

Bei dieser zuvor beschriebenen Ausführungsform der Erfindung ist es besonders vorteilhaft, dass der Einlegeboden nicht bewegt werden muss, sondern stillstehen kann, da das Inkubationsmedium mit Hilfe des von dem Rührmagnetantrieb angetriebenen Rührmagneten, ähnlich wie mit einem handelsüblichen Magnetrührer, bewegt werden kann.

Es sei aber darauf hingewiesen, dass eine mechanisch bewegbare Aufstellfläche mit wenigstens einem Rührmagnetantrieb an oder in dem Einlegeboden oder der Aufstellfläche des Einlegebodens kombinierbar ist und zu besonders guten Ergebnissen führen kann. Bei einer besonders vorteilhaften Ausführungsform der Erfindung kann der wenigstens eine Rührmagnetantrieb eine drehbar gelagerte Trägerscheibe mit wenigstens einem, bevorzugt zwei oder mehreren an der Trägerscheibe angeordneten Magneten und/oder Permanentmagneten aufweisen, wobei die Trägerscheibe mittels einer, beispielsweise der bereits erwähnten, Antriebsvorrichtung des Einlegebodens mechanisch antreibbar und in Rotation versetzbar ist, um ein umlaufendes Magnetfeld zu erzeugen, mit dem ein Rührmagnet in Bewegung versetzt werden kann.

Bei einem mechanisch angetriebenen Rührmagnetantrieb mit wenigstens einem Magneten und/oder Permanentmagnet kann vorteilhafterweise auf Magnetspulen verzichtet werden, die bei Betrieb Abwärme produzieren, die eine genaue Temperaturführung eines Inkubationsprozesses erschweren können.

Zweckmäßig kann es sein, wenn der wenigstens eine Rührmagnetantrieb dabei jeweils an einer Rührstelle und in Gebrauchsstellung unterhalb einer Aufstellfläche des Einlegebodens für wenigstens ein Inkubationsgefäß angeordnet ist.

Für die gleichzeitige Inkubationsbehandlung und Bewegung mehrerer Inkubationsgefäße kann es vorteilhaft sein, wenn der Einlegeboden und/oder eine, beispielsweise die bereits zuvor erwähnte Aufstellfläche des Einlegebodens eine Mehrzahl, bevorzugt gleichmäßig verteilt angeordneter, Rührmagnetantriebe zum Antrieb einer Mehrzahl in Inkubationsgefäße einsetzbarer Rührmagnete aufweist. Auch diese mehreren Rührmagnetantriebe können dann bevorzugt unterhalb einer oder der Aufstellfläche des Einlegebodens jeweils an einer Rührstelle angeordnet sein.

An dieser Stelle sei erwähnt, dass bei einer Ausführungsform des erfindungsgemäßen Einlegebodens vorgesehen sein kann, dass diese Mehrzahl, bevorzugt gleichmäßig verteilt angeordneter, Rührmagnetantriebe unabhängig voneinander steuer-/regelbar sein kann, um die Inkubationsmedien in den einzelnen Inkubationsgefäßen unterschiedlich stark in Bewegung versetzen zu können.

Zweckmäßig kann es sein, wenn die Aufstellfläche an einem mittels einer oder der Antriebsvorrichtung bewegbaren Schwingrahmen, insbesondere an dessen Gebrauchsstellung oberen Seite, angeordnet ist, wobei der Schwingrahmen relativ zu dem Rahmen des Einlegebodens bewegbar gelagert ist. So kann die bewegbare Aufstellfläche mit Hilfe des bewegbaren Schwingrahmens auf besonders einfache Art und Weise gegenüber dem in Gebrauchsstellung relativ zu dem Inkubator feststehenden Rahmen des Einlegebodens bewegt werden.

Vorteilhaft kann es sein, wenn der Schwingrahmen eine größere Dicke oder Höhe als der Rahmen aufweist und/oder so angeordnet ist, dass er in Gebrauchsstellung über den Rahmen des Einlegebodens nach oben übersteht. Auf diese Weise ist es möglich, dass eine an der Oberseite des Schwingrahmens angebrachte Aufstellfläche nicht mit dem den Schwingrahmen umgebenden feststehenden Rahmen des Einlegebodens während der Bewegung des Schwingrahmens und während der Bewegung der Aufstellfläche kollidiert. Zusätzlich oder alternativ dazu kann die relativ zu dem Rahmen bewegliche Aufstellfläche in einer zu der Oberfläche des Rahmens, insbesondere parallelen, beabstandeten Ebene angeordnet sein. Auf diese Weise kann sichergestellt werden, dass die Aufstellfläche während ihrer Bewegung relativ zu dem feststehenden Rahmen nicht mit diesem kollidiert und zumindest zeitweise sogar in eine den feststehenden Rahmen des Einlegebodens überlappende Stellung gebracht werden kann.

Vorteilhaft kann dies insbesondere dann sein, wenn die Aufstellfläche mit unterschiedlichen Bewegungsamplituden bewegt werden soll, um das in dem wenigstens einen Inkubationsgefäß befindliche Inkubationsmedium in Bewegung zu versetzen.

Bei einer Ausführungsform des erfindungsgemäßen Einlegebodens von besonderer Bedeutung kann vorgesehen sein, dass der Einlegeboden, insbesondere die Antriebsvorrichtung des Einlegebodens, einen Antriebsmotor aufweist, der bevorzugt als Elektromotor ausgebildet ist. Der Antriebsmotor kann, bevorzugt über die Antriebsvorrichtung, zumindest mittelbar mit der Aufstellfläche und/oder mit dem Schwingrahmen verbunden sein, um die Aufstellfläche und dadurch das Inkubationsmedium in Bewegung zu versetzen. Auf diese Weise wird ein Einlegeboden geschaffen, mit dem auch bestehende Inkubatoren oder Inkubationsschränke nachgerüstet werden können und somit durch eine weitere Funktion, nämlich die Möglichkeit, Inkubationsmedien während des Inkubationsvorgangs oder der Inkubationsbehandlung zu bewegen, aufgerüstet werden können.

Zweckmäßig kann es sein, wenn der Einlegeboden, insbesondere eine, beispielsweise die bereits zuvor erwähnte, Antriebsvorrichtung des Einlegebodens, einen Riementrieb aufweist, der den Schwingrahmen und/oder die Aufstellfläche des Einlegebodens und/oder den wenigstens einen Rührmagnetantrieb zumindest mittelbar mit einem, beispielsweise dem bereits zuvor erwähnten, Antriebsmotor verbindet und mittels dessen ein Antriebsmoment des Antriebsmotors auf die relativ zu dem Rahmen des Einlegebodens bewegliche Aufstellfläche und/oder auf den Schwingrahmen und/oder auf den wenigstens einen Rührmagnetantrieb übertragbar ist. Auf diese Weise können der Antriebsmotor an einer geeigneten Stelle auch in einer gewissen Distanz zu der Aufstellfläche bzw. zu dem Schwingrahmen oder auch zu dem wenigstens einen Rührmagnetantrieb angeordnet sein und diese Distanz zwischen dem Antriebsmotor und dem Schwingrahmen oder der bewegbaren Aufstellfläche mit Hilfe des Riementriebs überbrückt werden.

Zur Übertragung von Drehmomenten einerseits und gewünschten Bewegungsmustern andererseits kann es zweckmäßig sein, wenn der Einlegeboden, insbesondere die Antriebsvorrichtung des Einlegebodens, zum Bewegen der Aufstellfläche und/oder des Schwingrahmens eine mit einem, beispielsweise dem bereits zuvor erwähnten Antriebsmotors verbundene Abtriebsscheibe aufweist, die mit der Aufstellfläche und/oder dem Schwingrahmen verbunden und relativ zu dem feststehenden Rahmen des Einlegebodens drehbar gelagert ist. Dabei kann die Abtriebsscheibe vorzugsweise über einen, beispielsweise den bereits zuvor erwähnten, Riementrieb des Einlegebodens mit dem Antriebsmotor verbunden sein. Vorzugsweise kann die Abtriebsscheibe exzentrisch ausgebildet und/oder einen exzentrisch an der Abtriebsscheibe angeordneten Exzenterzapfen aufweisen. Dieser Exzenterzapfen kann zumindest mittelbar mit der Aufstellfläche verbunden sein und dazu dienen, eine Rotationsbewegung der Abtriebsscheibe in eine Exzenterbewegung des Schwingrahmens und/oder der Aufstellfläche des Einlegebodens umzusetzen. Je nach Exzentrizität der Abtriebsscheibe und/oder Anordnung des Exzenterzapfens kann das gewünschte Bewegungsmuster, beispielsweise eine kreisförmige Vibration oder eine Rotation, auf das wenigstens eine auf den Einlegeboden aufgestellte Inkubationsgefäß übertragen werden.

Damit der Einlegeboden eine gleichmäßige Wärmeverteilung innerhalb des Inkubators, in den er in Gebrauchsstellung eingeschoben oder eingesetzt ist, nicht ermöglicht, kann es zweckmäßig sein, wenn der Rahmen des Einlegebodens die Aufstellfläche und/oder den Schwingrahmen des Einlegebodens zumindest bereichsweise mit Abstand umfasst und/oder wenn der Einlegeboden wenigstens eine, bevorzugt eine Mehrzahl, insbesondere gleichmäßig verteilt an dem Einlegeboden angeordneter, den Einlegeboden durchsetzender Konvektionsöffnungen aufweist. Auf diese Weise ist es möglich, dass mittels des Inkubators erwärmte/erhitzte Luft durch den Abstand zwischen dem Rahmen des Einlegebodens und der Aufstellfläche bzw. dem Schwingrahmen hindurchströmen kann und somit eine gleichmäßige Wärmeverteilung in einem Innenraum des Inkubators ermöglicht. Gleiches gilt für einen Einlegeboden, der wenigstens eine den Einlegeboden durchsetzende Konvektionsöffnung aufweist, durch die dann ein Austausch und eine gleichmäßige Verteilung warmer Luft möglich sind.

Wenn der Einlegeboden an in Gebrauchsstellung des Einlegebodens einer Einschuböffnung des Inkubators zugewandten Seite wenigstens einen Griff und/oder wenigstens einen Tragegriff aufweist, kann der Einlegeboden einerseits einfach transportiert und andererseits einfach in und aus seiner Gebrauchsstellung in dem Inkubator eingeschoben bzw. herausgezogen oder eingesetzt und herausgenommen werden.

Zweckmäßig kann es zudem sein, wenn der Einlegeboden wenigstens einen Klemmgriff aufweist, mit dem der Einlegeboden in seiner in den Inkubator eingesetzten und/oder eingeschobenen Gebrauchsstellung festlegbar ist. Dabei kann dieser Klemmgriff mit einem an dem Einlegeboden vorgesehenen Klemmmechanismus verbunden sein, zu dessen Bedienung der Klemmgriff von einer Offenstellung in eine Klemmstellung gebracht werden kann. Mittels des Klemmmechanismus kann dann der Einlegeboden in seiner Gebrauchsstellung in bzw. an dem Inkubator fixiert werden.

Um den Einlegeboden auch mit bestehenden Inkubatoren oder Inkubationsschränken verwenden zu können, kann es zweckmäßig sein, wenn der Einlegeboden als Einschubboden ausgestaltet ist und in einen Inkubator, insbesondere in als Halteschienen ausgebildete Halteelemente eines Inkubators, einschiebbar ist. Um den Einlegeboden in seiner Gebrauchsstellung in dem Inkubator sicher festlegen zu können und so eine Relativbewegung zwischen dem Einlegeboden und dem Inkubator zu verhindern, kann es vorteilhaft sein, wenn der Einlegeboden wenigstens ein Zentrierelement, insbesondere wenigstens einen Zentrierzapfen und/oder wenigstens ein Zentrierloch, aufweist, wobei das wenigstens eine Zentrierelement des Einlegebodens dann mit einem korrespondierend ausgebildeten Zentrierelement des Inkubators zusammenwirken kann, um den Einlegeboden in eine vorbestimmte und durch die Zentrierelemente definierte Position zu bringen und in dieser zu halten.

Zweckmäßigerweise kann das wenigstens eine Zentrierelement des Einlegebodens dabei an einer Einschuböffnung des Inkubators in Gebrauchsstellung des Einlegebodens abgewandten Seite des Einlegebodens, also beispielsweise an seiner Rückseite, ausgebildet sein.

Um eine Bewegung des Einlegebodens, insbesondere seiner Aufstellfläche, zuverlässig auf das wenigstens eine in Gebrauchsstellung auf den Einlegeboden aufgestellte Inkubationsgefäß übertragen zu können, kann es zweckmäßig sein, wenn der Einlegeboden und/oder die Aufstellfläche des Einlegebodens wenigstens ein Halteelement für das wenigstens eine Inkubationsgefäß aufweist. Das wenigstens eine Halteelement kann dabei beispielsweise eine Noppenmatte und/oder ein Inkubationsgefäßhalter sein, der zur Sicherung wenigstens eines Inkubationsgefäßes auf der Aufstellfläche dient.

Ferner ist es möglich, dass der Einlegeboden und/oder die Aufstellfläche des Einlegebodens mit einer rutschhemmenden Beschichtung versehen sind. Dies kann zusätzlich oder alternativ vorgesehen sein.

Es sei erwähnt, dass eine Bewegung der beweglichen Aufstellfläche und/oder des Schwingrahmens des Einlegebodens und/oder eine auf das Inkubationsgefäß mittels des Einlegebodens übertragbare Bewegung eine Taumel- und/oder eine Kipp- und/oder eine Rotations- und/oder eine Schüttel- und/oder eine Rührbewegung sein kann. Während die Rührbewegung zweckmäßigerweise mittels eines Rührmagnetantriebes und eines mit dem Rührmagnetantrieb magnetisch gekoppelten Rührmagneten bewirkt wird, lassen sich die Taumel- und/oder eine Kipp- und/oder eine Rotations- und/oder eine Schüttelbewegung besonders gut mittels des Schwingrahmens und insbesondere mit Hilfe der zuvor beschriebenen Abtriebsscheibe realisieren.

Die zuvor beschriebene Aufgabe wird bei dem eingangs definierten Inkubator durch die Merkmale des Patentanspruchs 17 gelöst. Insbesondere wird die Aufgabe dadurch gelöst, dass der wenigstens eine Einlegeboden des Inkubators ein Einlegeboden nach einem der Ansprüche 1 bis 16 ist.

Dabei kann es zweckmäßig sein, wenn der Inkubator einen mit einer, beispielsweise der bereits zuvor erwähnten, Antriebsvorrichtung des wenigstens einen Einlegebodens koppelbaren Antriebsmotor aufweist. Dieser kann vorzugsweise als Elektromotor ausgebildet sein. Mit diesem Antriebsmotor kann eine, beispielsweise die bereits zuvor erwähnte, bewegliche Aufstellfläche des wenigstens einen in Gebrauchsstellung in den Inkubator eingesetzten und/oder eingeschobenen Einlegebodens bewegbar sein. Es ist aber auch möglich einen, beispielsweise den bereits zuvor erwähnten wenigstens einen, Rührmagnetantrieb des Einlegebodens mittels dieses Antriebsmotors anzutreiben.

Zweckmäßigerweise kann der Inkubator, insbesondere der Inkubationsraum des Inkubators und/oder ein Gehäuse des Inkubators, eine Öffnung, die bevorzugt als Einschuböffnung ausgebildet ist, zum Einschieben oder Einsetzen des wenigstens einen Einlegebodens aufweisen, wobei die Öffnung bevorzugt mittels einer Abdeckung und/oder einer Tür verschließbar ist.

Um den wenigstens einen Einlegeboden in unterschiedlichen Positionen im Inneren des Inkubationsraums des Inkubators anordnen zu können oder aber um mehrere Einlegeböden gleichzeitig in dem Inkubator anzuordnen, kann es zweckmäßig sein, wenn der Inkubator, insbesondere in seinem Inkubationsraum, eine Mehrzahl in Gebrauchsstellung vorzugsweise vertikal zueinander beabstandet angeordneter Halteelemente für den wenigstens einen Einlegeboden aufweist, mittels derer der wenigstens eine oder aber eine Mehrzahl an Einlegeböden in unterschiedlichen, übereinander angeordneten Haltestufen in dem Inkubationsraum anordenbar sind.

Die vertikal zueinander beabstandet angeordneten Halteelemente können dabei beispielsweise als Halteschienen, ähnlich denen eines Backofens, und/oder Halteösen ausgebildet sein, von denen der wenigstens eine Einlegeboden in Gebrauchsstellung formschlüssig gehalten werden kann. Es sei darauf hingewiesen, dass die Verwendung von Halteschienen bevorzugt ist, da so der wenigstens eine Einlegeboden besonders einfach in seine Gebrauchsstellung im Inneren des Inkubators eingeschoben werden kann.

Um den wenigstens einen Einlegeboden oder die Mehrzahl von Einlegeböden in dem Inkubator in ihrer Gebrauchsstellung sicher festlegen zu können, kann es zweckmäßig sein, wenn der Inkubator, insbesondere an einer Innenwand seines Inkubationsraums, wenigstens eine zu dem wenigstens einen Zentrierelement des Einlegebodens passend ausgebildetes und angeordnetes Zentrierelement aufweist. Dieses Zentrierelement des Inkubators ist dann selbstverständlich korrespondierend zu dem Zentrierelement des Einlegebodens ausgebildet. Das heißt, dass das Zentrierelement des Einlegebodens als Zentrierloch oder Zentrieröffnung ausgebildet ist, wenn das Zentrierelement des Einlegebodens als Zentrierzapfen ausgebildet ist. Wenn das Zentrierelement des Einlegebodens als Zentrierloch ausgebildet ist, ist das Zentrierelement des Inkubators korrespondierend dazu als in das Zentrierloch passender Zentrierzapfen ausgebildet.

Vorteilhaft kann es sein, wenn der Inkubator, insbesondere der Inkubationsraum und/oder eine Innenwand des Inkubators, in jeder der Gebrauchsstellung vertikal zueinander beabstandeten Haltestufen für den wenigstens einen Einlegeboden jeweils wenigstens ein zu dem Zentrierelement des Einlegebodens korrespondierend ausgebildetes Zentrierelement aufweist. So ist es möglich, den wenigstens einen Einlegeboden oder auch mehrere Einlegeböden in unterschiedlichen Haltestufen sicher in dem Inkubator zu positionieren.

Zweckmäßig kann es sein, wenn der mit der Antriebsvorrichtung des wenigstens einen Einlegebodens koppelbare Antriebsmotor an dem Inkubator, insbesondere an einem Gehäuse des Inkubators, angeordnet ist. Dabei kann ein Antriebsmoment des Antriebsmotors auf den wenigstens einen Einlegeboden, insbesondere auf die bewegliche Aufstellfläche und/oder auf den Schwingrahmen des Einlegebodens, vorzugsweise mittels eines, beispielsweise des bereits zuvor erwähnten Riementriebs, des Einlegebodens übertragbar sein. Bei dieser Ausführungsform des Inkubators ist besonders vorteilhaft und daher erwähnenswert, dass der Einlegeboden hier selbst keinen Antriebsmotor aufweisen muss und daher beispielsweise besser gereinigt werden kann, was insbesondere bei der Verarbeitung biologischer Proben häufig notwendig ist. Zudem kann dadurch verhindert werden, dass die durch den Betrieb des Antriebsmotors auftretende Wärmeabgabe bei der Temperaturführung des Inkubators berücksichtigt werden muss, da der Antriebsmotor hier außerhalb des Inkubationsraumes an dem Gehäuse des Inkubators angeordnet ist.

Zur Übertragung eines Drehmoments von einem, beispielsweise dem zuvor bereits erwähnten, Antriebsmotor des Inkubators auf den wenigstens einen Einlegeboden kann es vorteilhaft sein, wenn der wenigstens eine Einlegeboden eine Zapfwelle und der Inkubator für die Zapfwelle eine entsprechend angeordnete und ausgebildete Koppelstelle aufweisen, wobei an der Koppelstelle des Inkubators eine Abtriebswelle des Antriebsmotors mit der Zapfwelle des wenigstens einen Einlegebodens koppelbar ist. Auf diese Weise kann ein Drehmoment des Antriebsmotors des Inkubators, insbesondere mittels der Antriebsvorrichtung und/oder mittels des Riementriebs des wenigstens einen Einlegebodens, auf die bewegliche Aufstellfläche und/oder auf den Schwingrahmen und/oder auf den wenigstens einen Rührmagentantrieb des wenigstens einen Einlegebodens übertragen werden. Die Zapfwelle des Einlegebodens kann dabei insbesondere an einer der Einschuböffnung des Inkubators in den Inkubationsraum eingesetzter Gebrauchsstellung angewandten Seite oder Rückseite des Einlegebodens angeordnet sein.

Um den wenigstens einen Einlegeboden in mehreren der unterschiedlichen Haltestufen antreiben zu können, kann es zweckmäßig sein, wenn der Inkubator eine Mehrzahl passend zu den Haltestufen positionierter Koppelstellen für eine Zapfwelle des wenigstens einen Einlegebodens aufweist. Vorzugsweise kann der Inkubator einer Anzahl von Haltestufen entsprechende Anzahl passend zu den Haltestufen positionierter Koppelstellen aufweisen, um den wenigstens einen Einlegeboden über seine Zapfwelle in jeder der an dem Inkubator vorhandenen Haltestufen betreiben zu können.

Es ist aber auch möglich, dass der Antriebsmotor in einer Mehrzahl, bevorzugt einer Anzahl von Haltestufen entsprechenden Anzahl, zu Haltestufen passenden Positionen an dem Inkubator und/oder an einem Gehäuse des Inkubators anordenbar ist. Auf diese Weise kann der Antriebsmotor dann immer in die entsprechende Position an dem Inkubator gebracht werden, die zu der Haltestufe passt, in die der Einlegeboden in den Inkubator eingesetzt oder eingeschoben ist.

Es ist aber auch möglich, dass der Inkubator eine Mehrzahl von Antriebsmotoren, insbesondere eine der Anzahl von Haltestufen entsprechende Anzahl, passend zu den Haltestufen angeordneter Antriebsmotoren aufweist. Auf diese Weise kann an jeder der unterschiedlichen Haltestufen jeweils ein Antriebsmotor für einen entsprechend ausgerüsteten Einlegeboden vorhanden sein. Ferner ist es auf diese Weise möglich, einen Inkubator bereit zu stellen, bei dem auf unterschiedlichen Einlegeböden positionierte Inkubationsgefäße bzw. darin befindliche Inkubationsmedien zwar gleichzeitig, jedoch unabhängig voneinander, sei es mit unterschiedlichen Bewegungsmustern und/oder unterschiedlichen Bewegungsamplituden, sei es mit unterschiedlichen Bewegungsfrequenzen bzw. Bewegungsintensitäten, bewegt werden können.

Es sei darauf hingewiesen, dass bei einem einen Antriebsmotor aufweisenden Einlegeboden dieser Antriebsmotor mit einer externen Stromquelle für den Antriebsmotor verbunden sein kann. Es ist aber auch möglich, dass der Einlegeboden, insbesondere wenn er einen eigenen Antriebsmotor aufweist, einen Akkumulator und/oder eine Batterie als Stromquelle für den Antriebsmotor aufweist.

Nachfolgend sind Ausführungsbeispiele erfindungsgemäßer Einlegeböden und eines erfindungsgemäßen Inkubators anhand der Zeichnung näher erläutert. Es zeigen in teilweise stark schematisierter Darstellung:
- Fig. 1: Eine perspektivische Seitenansicht auf einen stark schematisiert dargestellten erfindungsgemäßen Inkubator mit einem in einen Inkubationsraum des Inkubators eingesetzten erfindungsgemäßen Einlegeboden, auf dem zwei Inkubationsgefäßhalter zu erkennen sind,
- Fig. 2: eine perspektivische Seitenansicht des in Fig. 1 dargestellten Inkubators, wobei eine Seitenwand des Inkubators der besseren Übersicht halber nicht dargestellt ist, so dass der Blick in den Innenraum des Inkubators frei und an der Rückseite des Inkubators ein im Schnitt dargestellter Antriebsmotors, der über eine Zapfwelle mit dem in Gebrauchsstellung befindlichen Einlegeboden koppelbar ist, zu erkennen sind,
- Fig. 3: eine perspektivische Rückansicht des in den Fig. 1 und 2 dargestellten Inkubators,
- Fig. 4: eine perspektivische Unteransicht des in den Fig. 1 und 2 dargestellten Einlegebodens, wobei an einer Rückseite des Einlegebodens eine Zapfwelle einer Antriebsvorrichtung des Einlegebodens zu erkennen ist, die in Gebrauchsstellung mit dem in den Fig. 2 und 3 dargestellten Antriebsmotors des Inkubators koppelbar ist,
- Fig. 5: eine Detaildarstellung des in den Fig. 1, 2 und 4 dargestellten Einlegebodens, in der die Zapfwelle und ein Riementrieb des Einlegebodens zu erkennen sind,
- Fig. 6: eine perspektivische Aufsicht auf einen weiteren erfindungsgemäßen Einlegeboden mit einer Zapfwelle an seiner Rückseite sowie mit drei die Aufstellfläche des Einlegebodens umgebenden Konvektionsöffnungen und mit zwei an der Rückseite des Einlegebodens angeordneten Zentrierzapfen,
- Fig. 7: eine perspektivische Draufsicht auf den in Fig. 6 dargestellten Einlegeboden, wobei die Aufstellfläche des Einlegebodens entfernt ist und ein Schwingrahmen sowie eine Abtriebsscheibe und der Riementrieb des Einlegebodens zu erkennen sind,
- Fig. 8: eine perspektivische Schnittansicht eines erfindungsgemäßen Einlegebodens, in der der Schwingrahmen, eine Abtriebsscheibe mit Exzenterzapfen und ein Flanschelement zu erkennen, sind, wobei der Exzenterzapfen in seiner Gebrauchsstellung in dem Flanschelement zur Übertragung seiner Bewegung auf die nicht dargestellte Aufstellfläche des Einlegebodens dargestellt ist,
- Fig. 9: eine perspektivische Unteransicht eines weiteren erfindungsgemäßen Einlegebodens, wobei an der Unterseite des Einlegebodens ein Antriebsmotor für die mechanisch bewegbare Aufstellfläche des Einlegebodens zu erkennen ist,
- Fig. 10: eine perspektivische Aufsicht auf einen weiteren erfindungsgemäßen Einlegeboden, der eine Mehrzahl von einer Aufstellfläche verdeckter Rührmagnetantriebe aufweist, sowie
- Fig. 11: eine perspektivische Ansicht des in Fig. 10 dargestellten erfindungsgemäßen Einlegebodens mit abgenommener Aufstellfläche, wobei insgesamt neun über einen Riementrieb miteinander verbundene Rührmagnetantriebe mit jeweils zwei Magneten zu erkennen sind, die über eine an der Rückseite des Einlegebodens angeordneten Zapfwelle und einen Riementrieb antreibbar sind.

Bei der nachfolgenden Beschreibung verschiedener Ausführungsformen der Erfindung erhalten in ihrer Funktion übereinstimmende Elemente auch bei abweichender Gestaltung oder Formgebung übereinstimmende Bezugszahlen.

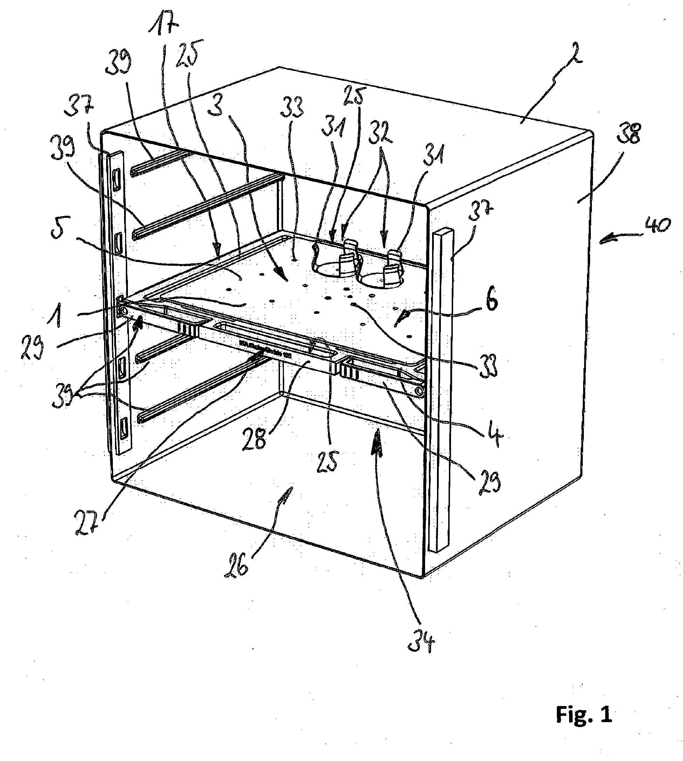

Die Fig. 1 und 2 sowie 4 bis 11 zeigen jeweils einen im Ganzen mit 1 bezeichneten Einlegeboden, der in Gebrauchsstellung in einem Inkubator 2 einsetzbar, insbesondere einschiebbar ist.

Der Einlegeboden 1 dient zur Aufnahme wenigstens eines in den Figuren nicht dargestellten Inkubationsgefäßes, in dem sich ein zu inkubierendes Inkubationsmedium befindet. Sämtliche der in den Figuren dargestellten Einlegeböden 1 sind mit wenigstens einem Mittel 3 versehen, mit dem ein Inkubationsmedium, dass sich in dem wenigstens einen auf den Einlegeboden 1 gestellten Inkubationsgefäß befindet, in Bewegung versetzbar ist.

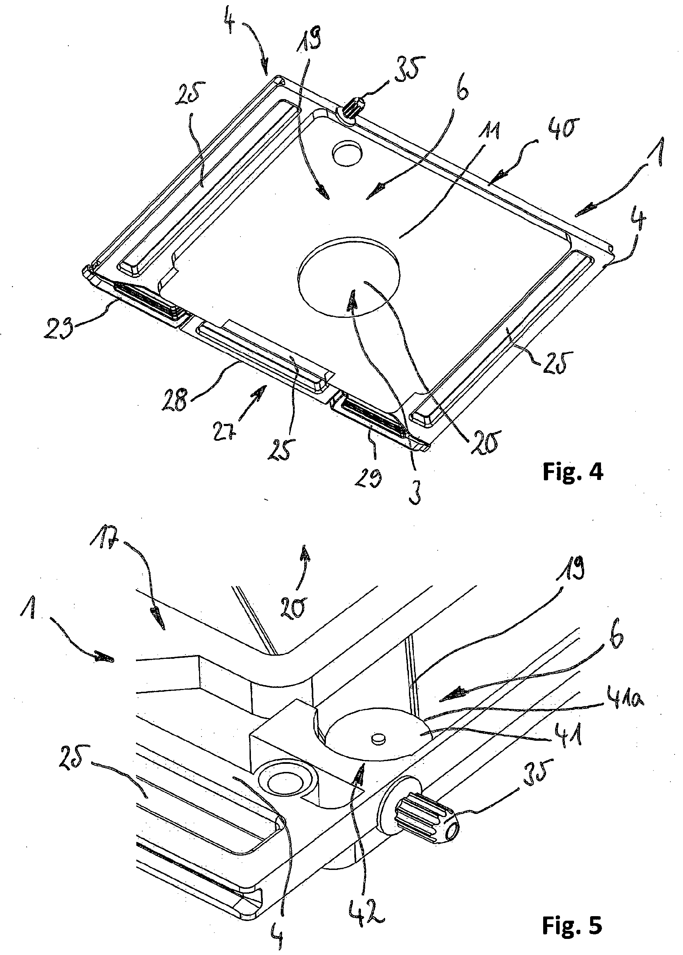

Das wenigstens eine Mittel 3 ist dabei elektrisch betreibbar und in Bezug auf einen in Gebrauchsstellung feststehenden Rahmen oder Einschubrahmen 4 des Einlegebodens 1 mechanisch bewegbar. Bei dem in dem Fig. 4 bis 9 dargestellten Einlegeboden 1 weist dieser als das wenigstens eine Mittel 3 zur Bewegung des Inkubationsmediums eine in zumindest einer Bewegungsrichtung bewegliche Aufstellfläche 5 zur Aufnahme wenigstens eines Inkubationsgefäßes und einen gegenüber der beweglichen Aufstellfläche 5 feststehenden Rahmen 4 auf, mit dem der Einlegeboden 1 in seiner in den Inkubator 2 eingesetzten Gebrauchsstellung festlegbar ist.

Dieser Rahmen 4 ist dabei als Einschubrahmen ausgebildet, mit dem der Einlegeboden 1 in den Inkubator 2 eingeschoben werden kann.

Der Einlegeboden 1 weist eine Antriebsvorrichtung 6 auf, mit der die bewegliche Aufstellfläche 5 des Einlegebodens 1 zumindest mittelbar bewegbar ist und mit der das in einem auf den Einlegeboden 1 gestelltes Inkubationsgefäß befindliche Inkubationsmedium zumindest mittelbar in Bewegung versetzbar ist.

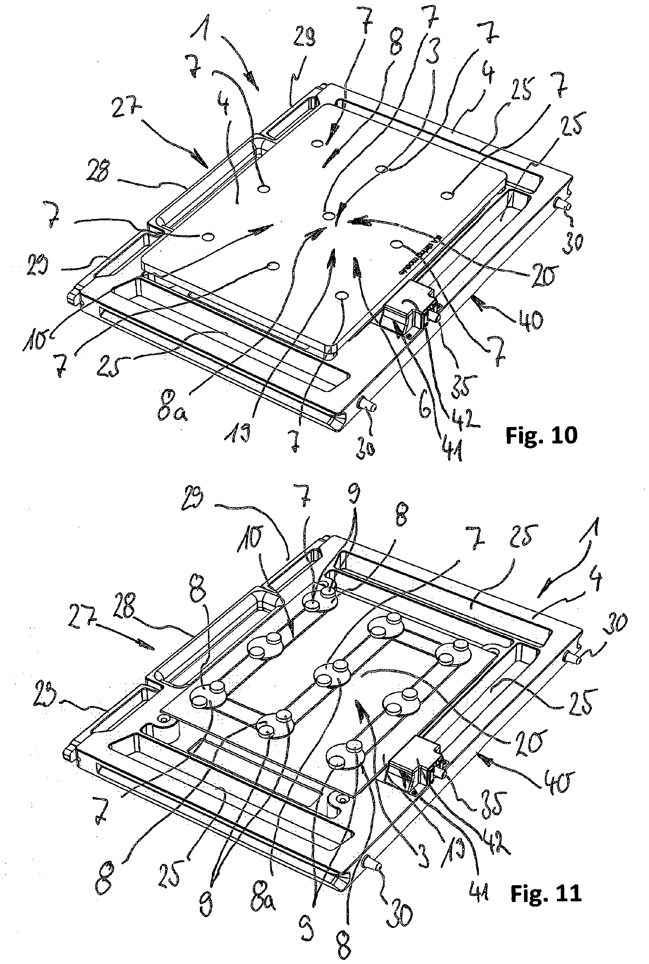

Der in den Fig. 10 und 11 dargestellte Einlegeboden 1 weist als Mittel 3 zur Bewegung des Inkubationsmediums insgesamt neun Rührmagnetantriebe 7 auf, die dazu eingerichtet sind, jeweils ein umlaufendes Magnetfeld zum Antrieb jeweils eines in ein Inkubationsgefäß einsetzbaren Rührmagneten zu erzeugen.

Fig. 11 zeigt dabei auch den Aufbau der verwendeten Rührmagnetantriebe 7.

Jeder der Rührmagnetantriebe 7 weist jeweils eine drehbar gelagerte Trägerscheibe 8 auf, an deren in Gebrauchsstellung oberen Seite jeweils zwei um 180° zueinander versetzt angeordnete Magnete oder Permanentmagnete 9 vorgesehen sind. Die Trägerscheiben 8 der Rührmagnetantriebe 7 sind, wie aus Fig. 11 ersichtlich, über einen gemeinsamen Antriebsriemen 10 miteinander verbunden. So ist es möglich, dass lediglich eine Trägerscheibe 8a der Trägerscheiben 8 der insgesamt neun Rührmagnetantriebe 7 angetrieben werden muss, da ein auf die die eine Trägerscheibe 8a von der Antriebsvorrichtung 6 übertragenes Drehmoment mittels des Antriebsriemens 10 auf die übrigen, über den Antriebsriemen 10 miteinander verbundenen Trägerscheiben 8 übertragen werden kann. Bei dem in den Fig. 10 und 11 dargestellten Ausführungsbeispiel des Einlegebodens 1 ist die mittlere, mit dem Bezugszeichen 8a versehener Trägerscheibe diejenige, die von der Antriebsvorrichtung 6 des Einlegebodens angetrieben werden kann.

Der in den Fig. 10 und 11 dargestellte Einlegeboden 1 weist unterhalb seiner Aufstellfläche 5 eine Mehrzahl gleichmäßig verteilt angeordneter Rührmagnetantriebe 7 zum Antrieb einer Mehrzahl in Inkubationsgefäße einsetzbarer Rührmagnete auf.

Bei den in den Fig. 1 und 2 sowie 4 bis 9 dargestellten Einlegeböden 1 ist die Aufstellfläche 5 an einem mittels der Antriebsvorrichtung 6 bewegbaren Schwingrahmen 11 an dessen in Gebrauchsstellung oberen Seite angeordnet, wobei der Schwingrahmen 11 relativ zu dem feststehenden Rahmen 4 des Einlegebodens 1 bewegbar gelagert ist.

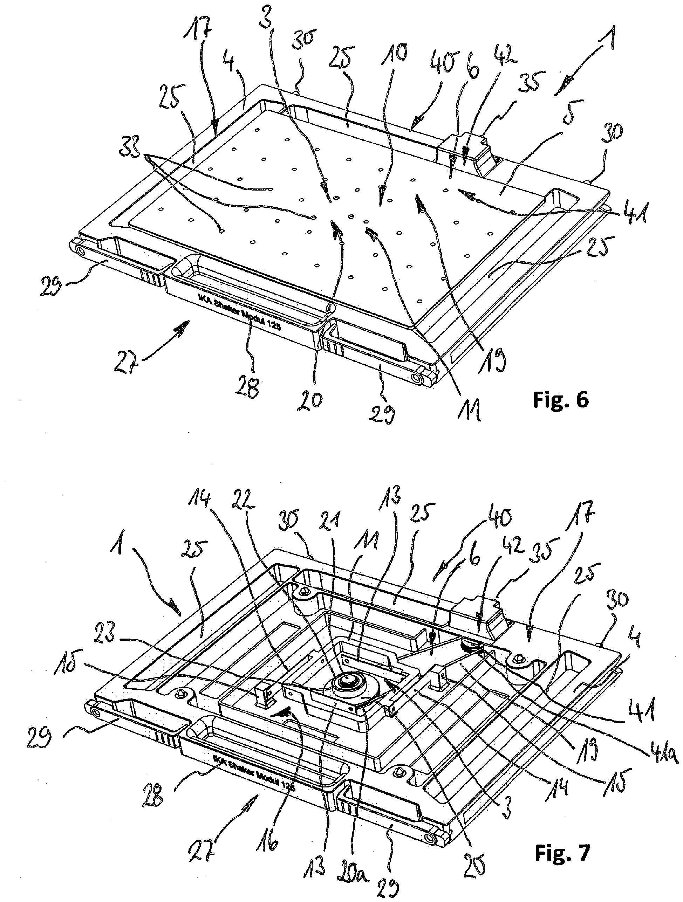

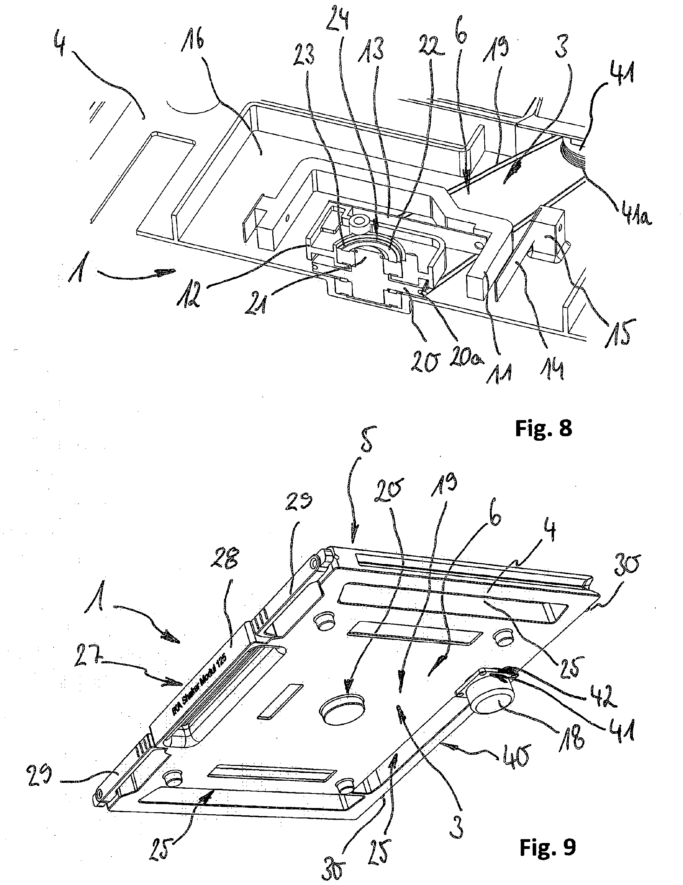

Der Schwingrahmen 11 ist dabei mehrteilig aufgebaut und weist ein Flanschelement 12 auf, an dem die Aufstellfläche 5 des Einlegebodens 1 in Gebrauchsstellung befestigt ist. Das Flanschelement 12 ist in Fig. 8 im Schnitt dargestellt. In Fig. 7, in der der Schwingrahmen 11 besonders gut zu erkennen ist, ist dieses Flanschelement 12 der besseren Übersicht halber weggelassen. Das Flanschelement 12 ist mittels eines inneren Paars Schwingfedern 13 gegenüber dem eigentlichen Schwingrahmen 11 beweglich gelagert. Der Schwingrahmen 11 und das Flanschelement 12 sind wiederum um ein zweites Paar äußerer Schwingfedern 14 gegenüber dem Einlegeboden 1 und seinem feststehenden Rahmen 4 beweglich gelagert. Aufgehängt ist der Schwingrahmen 11 mittels seines zweiten Paares Schwingfedern 14 an zwei Befestigungsköpfen 15, die an einer Grundplatte 16 des Einlegebodens in Gebrauchsstellung nach oben abstehend angeordnet sind.

Der Schwingrahmen 11 ist dabei so angeordnet, dass er in Gebrauchsstellung über den Rahmen 4 nach oben übersteht, so dass die relativ zu dem Rahmen 4 bewegliche Aufstellfläche 6 in einer zu der Oberfläche 17 des Rahmens 4 parallelen beabstandeten Ebene angeordnet ist.

Bei dem in Fig. 9 dargestellten Ausführungsbeispiel des Einlegebodens 1 weist der Einlegeboden 1 einen als Elektromotor ausgebildeten Antriebsmotor 18 auf, der über die Antriebsvorrichtung 6 zumindest mittelbar mit der Aufstellfläche 5 und so mittelbar mit dem Schwingrahmen 11 verbunden ist, um die Aufstellfläche 5 und dadurch das Inkubationsmedium in dem wenigstens einen auf die Aufstellfläche 5 in Gebrauchsstellung positionierten Inkubationsgefäß in Bewegung zu versetzen.

Die Fig. 5, 7 und 8 zeigen, dass der Einlegeboden 1 einen Riementrieb 19 aufweist, der den Schwingrahmen 11 und damit die Aufstellfläche 5 des Einlegebodens 1 zumindest mittelbar mit dem Antriebsmotor 18 verbindet und mittels dessen ein Antriebsmoment des Antriebsmotors 18 auf die relativ zu dem Rahmen 4 des Einlegebodens 1 bewegliche Aufstellfläche 5 und auf den Schwingrahmen 11 übertragbar ist.

Bei dem Ausführungsbeispiel des Einlegebodens 1 mit den Rührmagnetantrieben 7 gemäß Figuren 10 und 11 dient der Riementrieb 19 dazu, das Antriebsmoment des Antriebsmotors 18 auf die Trägerscheibe 8a der neun Trägerscheiben 8 zu übertragen.

Der Einlegeboden 1, insbesondere die Antriebsvorrichtung 6 des Einlegebodens 1 zum Bewegen der Aufstellfläche 5 und/oder des Schwingrahmens 11 eine über den Riementrieb 19 mit dem Antriebsmotor 18 verbundene Abtriebsscheibe 20 auf, die mit der Aufstellfläche 5 und mit dem Schwingrahmen 11 verbunden und relativ zu dem feststehenden Rahmen 4 des Einlegebodens 1 drehbar gelagert ist. An ihrem Umfang ist die Abtriebsscheibe 20 mit einer Nut 20a für den Riementrieb 19 versehen, in der der Riementrieb 19 sicher gehalten ist.

Der Riementrieb 19 läuft dabei zwischen der bereits erwähnten Abtriebsscheibe 20 und einer Eintriebsscheibe 41, die wiederum mit einer Getriebestufe 42 verbunden ist, über die das Drehmoment des Antriebsmotors 18 auf den Eintriebsscheibe 41, den Riementrieb 19 und schließlich auf die Abtriebsscheibe 20 und die der Abtriebsscheibe 20 nachgelagerten Elemente übertragen werden kann. Zur Aufnahme des Riementriebs 19 weist auch die Eintriebsscheibe 41 eine entsprechende Nut 41a auf.

An ihrer Oberseite weist die Abtriebsscheibe 20 einen Exzenterzapfen 21 auf, dessen Längsmittelachse parallel versetzt zu einer Rotationsachse der Abtriebsscheibe angeordnet ist. Auf den Exzenterzapfen 21 ist gemäß Fig. 8 ein Exzenterlager 22 aufgesetzt, dessen äußerer Lagerring 23 in Gebrauchsstellung in eine Lageraufnahme 24 des Flanschelements 12 eingreift. Auf diese Weise kann die Rotationsbewegung der Abtriebsscheibe 20 in eine exzentrische Orbital-Bewegung des Exzenterzapfens 21 und somit auch in eine exzentrische Orbital-Bewegung des Lagerrings 23 des Exzenterlagers 22 und schließlich auch der an dem Flanschelement 12 angebrachten Aufstellfläche 5 des Einlegebodens 1 umgesetzt werden.

Im Falle des in den Figuren 10 und 11 dargestellten Einlegebodens 1 mit den insgesamt neun gleichmäßig verteilt angeordneten Rührmagnetantrieben 7 sei darauf hingewiesen, dass hier die angetriebene Trägerscheibe 8a drehfest mit der Abtriebsscheibe 20 verbunden ist, um ein Drehmoment von dem Antriebsmotor 18 auch auf die acht übrigen Rührmagnetantriebe 7 mithilfe des die Rührmagnetantriebe 7 miteinander verbindenden Antriebsriemens 10 zu übertragen. Die drehfeste Verbindung zwischen der Trägerscheibe 7a und der Abtriebsscheibe 20 ist in den Figuren nicht dargestellt.

Die in den Figuren dargestellten Einlegeböden 1 weisen, je nach Ausführungsbeispiel insgesamt drei oder vier gleichmäßig verteilt an dem Einlegeboden 1 angeordnete, den Einlegeboden 1 durchsetzende Konvektionsöffnungen 25 auf, durch die von dem Inkubator 2 erwärmte Luft von einem Bereich unterhalb des Einlegebodens 1 in einen Bereich oberhalb des Einlegebodens 1 und umgekehrt strömen kann. Die Konvektionsöffnungen 25 können eine präzise Temperaturführung des Inkubationsprozessen auch bei eingesetzten Einlegeboden 1 in dem Inkubator 2 begünstigen.

Die Figuren zeigen, dass der Einlegeboden 1 an in Gebrauchsstellung des Einlegebodens 1 einer Einschuböffnung 26 des Inkubators 2 zugewandten Seite 27 einen Griffbereich in Form eines Tragegriffes 28 aufweist. Der Tragegriff 28 wird zudem von insgesamt zwei Klemmgriffen 29 flankiert, mittels derer der Einlegeboden 1 in seiner in den Inkubator 2 eingesetzten bzw. eingeschobenen Gebrauchsstellung festlegbar ist, indem die Klemmgriffe 29 in Klemmstellung mit jeweils einer Klemmleiste 37 an dem Inkubator 2 zusammenwirken.

Die in den Figuren dargestellten Einlegeböden 1 sind als Einschubböden ausgestaltet und in den Inkubator 2 in als Halteschienen 39 ausgebildete Haltelemente des Inkubators 2 einschiebbar.

Jeder der in den Figuren dargestellten Einlegeböden 1 weist an einer der Einschuböffnung 26 des Inkubators 2 in Gebrauchsstellung des Einlegebodens 1 abgewandten Seite 40 insgesamt 2 als Zentrierzapfen 30 ausgebildete Zentrierelemente auf, die in Gebrauchsstellung in ein an dem Inkubator 2 ausgebildetes Zentrierelement, hier ein Zentrierloch, eingreifen.

Gemäß Fig. 1 weist der Einlegeboden 1 an einer Oberseite seiner Aufstellfläche 5 insgesamt zwei, hier nur exemplarisch dargestellte Halteelemente 31 auf, die bei dem in Fig. 1 dargestellten Ausführungsbeispiel als Inkubationsgefäßhalter 32 ausgebildet sind. Diese als Inkubationsgefäßhalter 32 ausgebildeten Halteelemente 31 sind in gleichmäßig an der Oberseite der Aufstellfläche 5 verteilt angeordneten Befestigungslöchern 33 einsteckbar, so dass einerseits eine Mehrzahl an Haltelementen 31 an der Aufstellfläche 5 angeordnet werden können und andererseits eine Formation der Halteelemente 31 an der Oberseite der Aufstellfläche 5 in Grenzen frei wählbar ist.

In den Figuren ist nicht dargestellt, dass die Aufstellfläche 5 des Einlegebodens 1 zur Sicherung wenigstens eines Inkubationsgefäßes auf der Aufstellfläche 5 zudem mit einer rutschhemmenden Beschichtung und/oder mit einer Noppenmatte als rutschhemmende Auflage versehen sein kann.

Die Bewegung, die die bewegliche Aufstellfläche 5 und der Schwingrahmen 11 des Einlegebodens 1 und damit eine auf das Inkubationsmedium übertragbare Bewegung ist eine Rotationsbewegung.

Der in den Fig. 1 bis 3 dargestellte Inkubator 2 weist einen beheizbaren Inkubationsraum 34 auf. Der in den Fig. 1 bis 3 dargestellt Inkubator 2 weist zudem einen mit der Antriebsvorrichtung 6 des Einlegebodens 1 koppelbaren, als Elektromotor ausgebildeten Antriebsmotor 18 auf, mit dem die bewegliche Aufstellfläche 5 des in Gebrauchsstellung in den Inkubator 2 eingesetzten oder eingeschobenen Einlegebodens 1 bewegbar ist.

Den Antriebsmotor 18 dabei an dem Inkubator 2 und nicht direkt an dem Einlegeboden 1 vorzusehen, stellt die bevorzugte Ausführungsform der Erfindung dar, da so vermieden werden kann, dass mit dem Antriebsmotor 18 eine zusätzliche Wärmequelle im Inneren des Inkubators 2 bzw. des Inkubationsraums 34 angeordnet ist, die eine genaue Temperaturführung während eines Inkubationsvorganges erschweren kann.

Die Einschuböffnung 26 des Inkubators 2, durch die einerseits der wenigstens eine Einlegeboden 1 in den Inkubationsraum 34 eingebracht werden kann und durch die andererseits der Inkubationsraum 34 zugänglich ist, ist mittels einer in den Figuren nicht dargestellten Abdeckung oder einer Tür verschließbar, um die in dem Inkubationsraum 34 des Inkubators 3 eingestellte Temperatur aufrecht erhalten zu können.

Wie bereits zuvor beschrieben, weist der Inkubator 2 in seinem Inkubationsraum 34 eine Mehrzahl in Gebrauchsstellung vertikal zueinander beabstandet angeordneter, einzelne Haltestufen definierende Halteelemente auf, die bei dem in den Figuren dargestellten Inkubator 2 als Halteschienen 39 ausgebildet sind. Die Halteschienen 39 ermöglichen dabei, dass wenigstens ein Einlegeboden 1 bzw. eine Mehrzahl von Einlegeböden 1 gleichzeitig in unterschiedlichen, übereinander angeordneten Haltestufen in dem Inkubationsraum 34 angeordnet werden können.

Der Inkubator 2 weist außerdem an einer Innenwand seines Inkubationsraumes 34 zwei zu den beiden Zentrierzapfen 30 passend ausgebildete Zentrierelemente, hier Zentrierlöcher, auf, die in den Figuren nicht dargestellt sind.

In den Figuren nicht dargestellt ist, dass der Inkubator 2 in jeder der in Gebrauchsstellung vertikal zueinander beabstandeten Haltestufen für den wenigstens einen Einlegeboden 1 jeweils zwei zu den beiden Zentrierelementen 30 des Einlegebodens 1 korrespondierend ausgebildete Zentrierelemente aufweist.

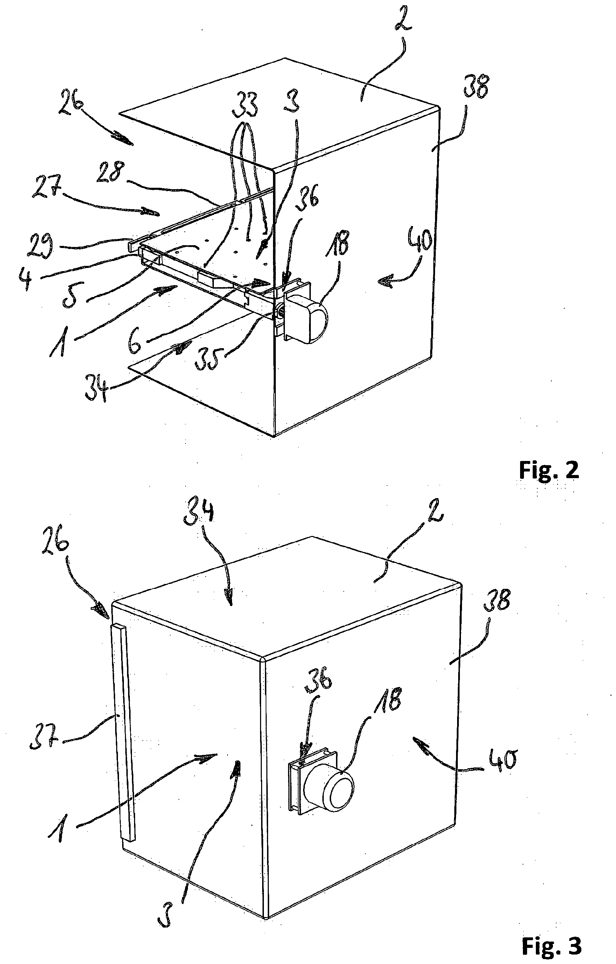

Wie bereits zuvor erwähnt, ist der mit der Antriebsvorrichtung 6 des wenigstens einen Einlegebodens 1 koppelbare Antriebsmotor 18 an dem Inkubator 2 angeordnet. Die Fig. 2 und 3 zeigen, dass der Antriebsmotor 18 dabei an einer Rückseite eines Gehäuses 38 des Inkubators 2 angeordnet ist, wobei ein Antriebsmoment dieses Antriebsmotors 18 auf den wenigstens einen Einlegeboden 1, insbesondere auf die bewegliche Aufstellfläche 5 und/oder auf den Schwingrahmen 11 oder aber auch auf die einzelnen Rührmagnetantriebe 7 mittels des Riementriebs 19 des Einlegebodens 1 übertragbar ist.

Die Ausführungsbeispiele des Einlegebodens 1 gemäß den Fig. 4, 5, 6, 7 sowie 10 und 11 zeigen ferner, dass der Einlegeboden 1 an einer der Einschuböffnung 26 des Inkubators 2 in den Inkubationsraum 34 eingesetzter Gebrauchsstellung abgewandten Seite 40 eine Zapfwelle 35 und der Inkubator 2 für die Zapfwelle 35 eine entsprechend angeordnete Koppelstelle 36 aufweisen, wobei an der Koppelstelle 36 des Inkubators 2 eine Abtriebswelle des Antriebsmotors 18 (in den Figuren nicht dargestellt) mit der Zapfwelle 35 des Einlegebodens 1 koppelbar ist.

Mithilfe der Zapfwelle 35 kann ein Drehmoment des Antriebsmotors 18 über eine Getriebestufe 42 auf die Eintriebsscheibe 41 und von dort auf den Riementrieb 19 übertragen werden.

Bei einem in den Figuren nicht dargestellten Ausführungsbeispiel des Inkubators 2 weist der Inkubator 2 eine Mehrzahl, bevorzugt einer Anzahl von Haltestufen für den wenigstens einen Einlegeboden 1 entsprechende Anzahl, passend zu den Haltestufen positionierter Koppelstellen 36 für eine Zapfwelle 35 des wenigstens einen Einlegebodens 1 auf.

Bei einer wiederum anderen Ausführungsform des Inkubators 2 ist außerdem vorgesehen, dass der Antriebsmotor 18 in einer Mehrzahl, bevorzugt in einer Anzahl von Haltestufen entsprechenden Anzahl, zu Haltestufen passenden Positionen an dem Inkubator 2 bzw. an seinem Gehäuse 38 anordenbar ist.

Bei einer dritten, ebenfalls in den Figuren nicht dargestellten Ausführungsform des Inkubators 2 ist vorgesehen, dass der Inkubator 2 eine Mehrzahl von Antriebsmotoren 18, insbesondere eine der Anzahl von Haltestufen entsprechende Anzahl von Antriebsmotoren 18 aufweist, die passend zu den Haltestufen des Inkubators 2 an dem Inkubator 2 angeordnet sind und jeweils einen passend zu der Zapfwelle 35 des wenigstens einen Einlegebodens 1 ausgebildete Koppelstellen aufweisen.

Der Einlegeboden 1 zeichnet sich dadurch aus, dass dieser wenigstens ein Mittel 3 aufweist, mit dem ein in einem auf den Einlegeboden 1 aufgestelltes Inkubationsgefäß befindliches Inkubationsmedium während einer Inkubationsbehandlung oder während eines Inkubationsprozesses innerhalb des Inkubators 2 in Bewegung versetzt werden kann. Dazu kann das wenigstens eine Mittel 3 elektrisch betrieben und/oder vorzugsweise mechanisch bewegt werden.

## Patentansprüche

1. Einlegeboden (1) zur Aufnahme wenigstens eines Inkubationsgefäßes, wobei der Einlegeboden (1) in einen Inkubator (2) einsetzbar, insbesondere einschiebbar, und in Gebrauchsstellung in den Inkubator (2) eingesetzt und auswechselbar ist, und wobei der Einlegeboden (1) mit wenigstens einem Mittel (3) versehen ist, mit dem ein Inkubationsmedium, das sich in dem auf den Einlegeboden (1) gestellten Inkubationsgefäß befindet, in Bewegung versetzbar ist, **dadurch gekennzeichnet, dass**
a) das Mittel zur Bewegung des Inkubationsmediums einen Rührmagnetantrieb aufweist, wobei der Einlegeboden (1) eine Antriebsvorrichtung (6) aufweist, mit der das in einem auf den Einlegeboden (1) gestellten Inkubationsgefäß befindliche Inkubationsmedium zumindest mittelbar in Bewegung versetzbar ist, und/oder
b) dass der Einlegeboden (1) als wenigstens ein Mittel (3) zur Bewegung des Inkubationsmediums eine in zumindest einer Bewegungsrichtung bewegliche Aufstellfläche (5) zur Aufnahme wenigstens eines Inkubationsgefäßes und einen gegenüber der beweglichen Aufstellfläche (5) feststehenden Rahmen (4) aufweist, mit dem der Einlegeboden (1) in seiner in den Inkubator (2) eingesetzten Gebrauchsstellung festlegbar ist, wobei der Einlegeboden (1) eine Antriebsvorrichtung (6) aufweist, mit der die bewegliche Aufstellfläche (5) des Einlegebodens (1) zumindest mittelbar bewegbar ist.

2. Einlegeboden (1) nach Anspruch 1 **dadurch gekennzeichnet, dass** das wenigstens eine Mittel (3) elektrisch betreibbar und/oder, vorzugsweise in Bezug auf einen in Gebrauchsstellung feststehenden Rahmen oder Einschubrahmen (4) des Einlegebodens (1), mechanisch bewegbar ist.

3. Einlegeboden (1) nach Anspruch 1 oder 2, **dadurch gekennzeichnet, dass** der feststehende Rahmen (4) ein Einschubrahmen ist.

4. Einlegeboden (1) nach einem der Ansprüche 2 bis 3, **dadurch gekennzeichnet, dass** der Rührmagnetantrieb (7) dazu eingerichtet ist, ein umlaufendes Magnetfeld zum Antrieb wenigstens eines in ein Inkubationsgefäß einsetzbaren Rührmagneten zu erzeugen, insbesondere wobei der wenigstens eine Rührmagnetantrieb (7) mittels einer oder der Antriebsvorrichtung (6) des Einlegebodens (1) antreibbar ist.

5. Einlegeboden (1) nach einem der Ansprüche 2 bis 4, **dadurch gekennzeichnet, dass** der wenigstens eine Rührmagnetantrieb (7) eine drehbar gelagerte Trägerscheibe (8, 8a) mit wenigstens einem, bevorzugt zwei oder mehreren an der Trägerscheibe (8, 8a) angeordneten Magneten und/oder Permanentmagneten (9) aufweist, vorzugsweise wobei die Trägerscheibe (8, 8a) mittels der Antriebsvorrichtung (6) des Einlegebodens (1) mechanisch antreibbar und in Rotation versetzbar ist.

6. Einlegeboden (1) nach einem der Ansprüche 1 bis 5, **dadurch gekennzeichnet, dass** der Einlegeboden (1) und/oder die Aufstellfläche (5) des Einlegebodens (1) eine Mehrzahl, bevorzugt gleichmäßig verteilt angeordneter, Rührmagnetantriebe (7) zum Antrieb einer Mehrzahl in Inkubationsgefäße einsetzbarer Rührmagnete aufweist.

7. Einlegeboden (1) nach einem der Ansprüche 1 bis 6, **dadurch gekennzeichnet, dass** die Aufstellfläche (5) an einem mittels der Antriebsvorrichtung (6) bewegbaren Schwingrahmen (11), insbesondere an dessen in Gebrauchsstellung oberen Seite, angeordnet ist, wobei der Schwingrahmen (11) relativ zu dem Rahmen (4) des Einlegebodens (1) bewegbar gelagert ist.

8. Einlegeboden (1) nach einem der Ansprüche 1 bis 7, **dadurch gekennzeichnet, dass** der Schwingrahmen (11) eine größere Dicke oder Höhe als der Rahmen (4) aufweist und/oder so angeordnet ist, dass er in Gebrauchsstellung über den Rahmen (4) nach oben übersteht und/oder dass die relativ zu dem Rahmen (4) bewegliche Aufstellfläche (6) in einer zu der Oberfläche (17) des Rahmens (4), insbesondere parallelen, beabstandeten Ebene angeordnet ist.

9. Einlegeboden (1) nach einem der Ansprüche 1 bis 8, **dadurch gekennzeichnet, dass** der Einlegeboden (1), insbesondere die Antriebsvorrichtung (6) des Einlegebodens (1), einen, bevorzugt als Elektromotor ausgebildeten, Antriebsmotor (18) aufweist, der, bevorzugt über die Antriebsvorrichtung (6), zumindest mittelbar mit der Aufstellfläche (5) und/oder mit dem Schwingrahmen (11) und/oder mit einem oder dem wenigstens einen Rührmagnetantrieb (7) verbunden ist, um die Aufstellfläche (5) und/oder das Inkubationsmedium in Bewegung zu versetzen.

10. Einlegeboden (1) nach einem der Ansprüche 1 bis 9, **dadurch gekennzeichnet, dass** der Einlegeboden (1), insbesondere die Antriebsvorrichtung (6) des Einlegebodens (1), einen Riementrieb (19) aufweist, der der den Schwingrahmen (11) und/oder die Aufstellfläche (15) des Einlegebodens (1) und/oder einen oder den wenigstens einen Rührmagnetantrieb (7) zumindest mittelbar mit einem oder dem Antriebsmotor (18) verbindet und mittels dessen ein Antriebsmoment des Antriebsmotors (18) auf die relativ zu dem Rahmen (4) des Einlegebodens (1) bewegliche Aufstellfläche (5) und/oder auf den Schwingrahmen (11) und/oder auf einen oder den wenigstens einen Rührmagnetantrieb (7) übertragbar ist.

11. Einlegeboden (1) nach einem der Ansprüche 1 bis 10, **dadurch gekennzeichnet, dass** der Einlegeboden (1), insbesondere die Antriebsvorrichtung (6) des Einlegebodens (1), zum Bewegen der Aufstellfläche und/oder des Schwingrahmens (11) und/oder eines oder des wenigstens einen Rührmagnetantriebs (7) eine, bevorzugt über einen oder den Riementrieb (19), mit einem oder dem Antriebsmotor (18) verbundene Abtriebsscheibe (20) aufweist, die mit der Aufstellfläche (5) und/oder mit dem Schwingrahmen (11) und/oder mit dem wenigstens einen Rührmagnetantrieb (7) zumindest mittelbar verbunden und relativ zu einem oder dem feststehenden Rahmen (4) des Einlegebodens (1) drehbar gelagert ist, und/oder dass die Abtriebsscheibe (20) exzentrisch ausgebildet ist und/oder einen exzentrisch an der Abtriebsscheibe (20) angeordneten Exzenterzapfen (21) aufweist, der zumindest mittelbar mit der Aufstellfläche (6) verbunden ist und mittels dessen eine Rotationsbewegung der Abtriebsscheibe (20) in eine Exzenterbewegung des Schwingrahmens (11) und/oder der Aufstellfläche (6) umsetzbar ist.

12. Einlegeboden (1) nach einem der Ansprüche 1 bis 11, **dadurch gekennzeichnet, dass** der Rahmen (4) des Einlegebodens (1) die Aufstellfläche (5) und/oder den Schwingrahmen (11) des Einlegebodens (1) zumindest bereichsweise mit Abstand umfasst und/oder dass der Einlegeboden (1) wenigstens eine, bevorzugt eine Mehrzahl, insbesondere gleichmäßig verteilt an dem Einlegeboden (1) angeordneter, den Einlegeboden (1) durchsetzende Konvektionsöffnung(en) (25) aufweist.

13. Einlegeboden (1) nach einem der Ansprüche 1 bis 12, **dadurch gekennzeichnet, dass** der Einlegeboden (1) an einer in Gebrauchsstellung des Einlegebodens (1) einer Einschuböffnung (26) des Inkubators (2) zugewandten Seite (27) wenigstens einen Griffbereich und/oder wenigstens einen Tragegriff (28) und/oder wenigstens einen Klemmgriff (29) aufweist, mit dem der Einlegeboden (1) in seiner in den Inkubator (2) eingesetzten und/oder eingeschobenen Gebrauchsstellung festlegbar ist.

14. Einlegeboden (1) nach einem der Ansprüche 1 bis 13, **dadurch gekennzeichnet, dass** der Einlegeboden (1) als Einschubboden ausgestaltet ist und in einen Inkubator (2), insbesondere in als Halteschienen (39) ausgebildete Haltelemente eines Inkubators (2), einschiebbar ist und/oder dass der Einlegeboden (1), insbesondere an einer Einschuböffnung (26) des Inkubators (2) in Gebrauchsstellung des Einlegebodens (1) abgewandten Seite (40), wenigstens ein Zentrierelement, insbesondere wenigstens einen Zentrierzapfen (30) und/oder wenigstens ein Zentrierloch, aufweist.

15. Einlegeboden (1) nach einem der Ansprüche 1 bis 14, **dadurch gekennzeichnet, dass** der Einlegeboden (1) und/oder die Aufstellfläche (5) des Einlegebodens (1) wenigstens ein Halteelement (31), insbesondere eine Noppenmatte und/oder ein Inkubationsgefäßhalter (32), zur Sicherung wenigstens eines Inkubationsgefäßes auf der Aufstellfläche (5) aufweist und/oder mit einer rutschhemmenden Beschichtung versehen ist.

16. Inkubator (2) mit einem beheizbaren Inkubationsraum (34) und mit wenigstens einem in Gebrauchsstellung in den Inkubationsraum (34) des Inkubators (2) eingesetzten und/oder eingeschobenen, auswechselbaren Einlegeboden (1) zur Aufnahme wenigstens eines, ein Inkubationsmedium enthaltenden Inkubationsgefäßes, **dadurch gekennzeichnet, dass** der wenigstens eine Einlegeboden (1) ein Einlegeboden (1) nach einem der Ansprüche 1 bis 15 ist.

17. Inkubator (2) nach Anspruch 16, **dadurch gekennzeichnet, dass** der Inkubator (2) einen mit einer oder der Antriebsvorrichtung (6) des wenigstens einen Einlegebodens (1) koppelbaren, bevorzugt als Elektromotor ausgebildeten, Antriebsmotor (18) aufweist, mit dem eine oder die bewegliche Aufstellfläche (5) des wenigstens einen in Gebrauchsstellung in den Inkubator (2) eingesetzten und/oder eingeschobenen Einlegebodens (1) bewegbar ist.

18. Inkubator (2) nach einem der Ansprüche 16 bis 17, **dadurch gekennzeichnet, dass** der mit der Antriebsvorrichtung (6) des wenigstens einen Einlegebodens (1) koppelbare Antriebsmotor (18) an dem Inkubator (2), insbesondere an einem Gehäuse (38) des Inkubators (2), angeordnet ist und dass ein Antriebsmoment des Antriebsmotors (18) auf den wenigstens einen Einlegeboden (1), insbesondere auf die bewegliche Aufstellfläche (5) und/oder auf den Schwingrahmen (11), vorzugsweise mittels eines oder des Riementriebs (19) des Einlegebodens (1), übertragbar ist.

## Claims

1. Insert shelf (1) for holding at least one incubation vessel, wherein the insert shelf (1) is able to be inserted, in particular able to be slid, into an incubator (2) and, in a position of use, is inserted into the incubator (2) and is exchangeable, and wherein the insert shelf (1) is provided with at least one means (3) by way of which an incubation medium situated in the incubation vessel placed on the insert shelf (1) is able to be set in motion,
**characterized in that**
a) the means for moving the incubation medium has a stirring-magnet drive, wherein the insert shelf (1) has a drive device (6) by way of which the incubation medium situated in an incubation vessel placed on the insert shelf (1) is at least indirectly able to be set in motion, and/or
b) **in that** the insert shelf (1) has, as at least one means (3) for moving the incubation medium, a set-up surface (5), which is movable in at least one movement direction and serves for holding at least one incubation vessel, and a frame (4), which in comparison with the movable set-up surface (5) is stationary and by way of which the insert shelf (1) is fixable in its position of use inserted into the incubator (2), wherein the insert shelf (1) has a drive device (6) by way of which the movable set-up surface (5) of the insert shelf (1) is at least indirectly movable.

2. Insert shelf (1) according to Claim 1, **characterized in that** the at least one means (3) is electrically operable and/or, preferably in relation to a frame or slide-in frame (4), stationary in the position of use, of the insert shelf (1), mechanically movable.

3. Insert shelf (1) according to Claim 1 or 2, **characterized in that** the stationary frame (4) is a slide-in frame.

4. Insert shelf (1) according to either of Claims 2 and 3, **characterized in that** the stirring-magnet drive (7) is configured to generate a rotating magnetic field for driving at least one stirring magnet which is insertable into an incubation vessel, in particular wherein the at least one stirring-magnet drive (7) is able to be driven by means of a or the drive device (6) of the insert shelf (1) .

5. Insert shelf (1) according to one of Claims 2 to 4, **characterized in that** the at least one stirring-magnet drive (7) has a rotatably mounted carrier disc (8, 8a) having at least one, preferably two or more magnets and/or permanent magnets (9) arranged on the carrier disc (8, 8a), preferably wherein the carrier disc (8, 8a) is able to be driven mechanically, and is able to be set in rotation, by means of the drive device (6) of the insert shelf (1).

6. Insert shelf (1) according to one of Claims 1 to 5, **characterized in that** the insert shelf (1) and/or the set-up surface (5) of the insert shelf (1) have/has a plurality of stirring-magnet drives (7) for driving a plurality of stirring magnets which are insertable into incubation vessels, which stirring-magnet drives are preferably arranged in a uniformly distributed manner.

7. Insert shelf (1) according to one of Claims 1 to 6, **characterized in that** the set-up surface (5) is arranged on a swinging frame (11) which is movable by means of the drive device (6), in particular is arranged on that side of said swinging frame which is the upper side in the position of use, wherein the swinging frame (11) is mounted so as to be movable relative to the frame (4) of the insert shelf (1).

8. Insert shelf (1) according to one of Claims 1 to 7, **characterized in that** the swinging frame (11) has a greater thickness or height than the frame (4) and/or is arranged in such a way that, in the position of use, it projects upwards beyond the frame (4), and/or **in that** the set-up surface (5), which is movable relative to the frame (4), is arranged in an plane which is spaced apart from the surface (17) of the frame (4) and is in particular parallel thereto.

9. Insert shelf (1) according to one of Claims 1 to 8, **characterized in that** the insert shelf (1), in particular the drive device (6) of the insert shelf (1), has a drive motor (18), preferably in the form of an electric motor, which, preferably via the drive device (6), is at least indirectly connected to the set-up surface (5) and/or to the swinging frame (11) and/or to a or the at least one stirring-magnet drive (7) in order to set the set-up surface (5) and/or the incubation medium in motion.

10. Insert shelf (1) according to one of Claims 1 to 9, **characterized in that** the insert shelf (1), in particular the drive device (6) of the insert shelf (1), has a belt drive (19) which at least indirectly connects the swinging frame (11) and/or the set-up surface (5) of the insert shelf (1) and/or a or the at least one stirring-magnet drive (7) to a or the drive motor (18) and by means of which a drive torque of the drive motor (18) is able to be transmitted to the set-up surface (5), movable relative to the frame (4) of the insert shelf (1), and/or to the swinging frame (11) and/or to a or the at least one stirring-magnet drive (7).

11. Insert shelf (1) according to one of Claims 1 to 10, **characterized in that**, for the purpose of moving the set-up surface and/or the swinging frame (11) and/or a or the at least one stirring-magnet drive (7), the insert shelf (1), in particular the drive device (6) of the insert shelf (1), has a drive output disc (20) which is connected, preferably via a or the belt drive (19), to a or the drive motor (18) and which is at least indirectly connected to the set-up surface (5) and/or to the swinging frame (11) and/or to the at least one stirring-magnet drive (7) and which is mounted so as to be rotatable relative to a or the stationary frame (4) of the insert shelf (1), and/or **in that** the drive output disc (20) is of eccentric form and/or has an eccentric peg (21) which is arranged eccentrically on the drive output disc (20) and which is at least indirectly connected to the set-up surface (5) and by means of which a rotational movement of the drive output disc (20) is able to be converted into an eccentric movement of the swinging frame (11) and/or the set-up surface (5).

12. Insert shelf (1) according to one of Claims 1 to 11, **characterized in that** the frame (4) of the insert shelf (1) surrounds the set-up surface (5), and/or the swinging frame (11) of the insert shelf (1), with a spacing at least regionally, and/or **in that** the insert shelf (1) has at least one, preferably a plurality of convection opening(s) (25) arranged in the insert shelf (1) in particular in a uniformly distributed manner and passing through the insert shelf (1).

13. Insert shelf (1) according to one of Claims 1 to 12, **characterized in that** the insert shelf (1) has on a side (27) which, in the position of use of the insert shelf (1), faces towards a slide-in opening (26) of the incubator (2) at least one gripping region and/or at least one carrying handle (28) and /or at least one clamping grip (29) by way of which the insert shelf (1) is fixable in its position of use inserted and/or slid into the incubator (2).

14. Insert shelf (1) according to one of Claims 1 to 13, **characterized in that** the insert shelf (1) is in the form of a slide-in shelf and is able to be slid into an incubator (2), in particular into holding elements, in the form of holding rails (39), of an incubator (2), and/or **in that** the insert shelf (1), in particular on a side (40) facing away from a slide-in opening (26) of the incubator (2) in the position of use of the insert shelf (1), has at least one centring element, in particular at least one centring peg (30) and/or at least one centring hole.

15. Insert shelf (1) according to one of Claims 1 to 14, **characterized in that** the insert shelf (1) and/or the set-up surface (5) of the insert shelf (1) have/has at least one holding element (31), in particular a pimpled mat and/or an incubation-vessel holder (32), for securing at least one incubation vessel on the set-up surface (5) and/or are/is provided with an anti-slip coating.

16. Incubator (2) having a heatable incubation chamber (34) and having at least one exchangeable insert shelf (1) which, in a position of use, is inserted and/or slid into the incubation chamber (34) of the incubator (2) and which serves for holding at least one incubation vessel containing an incubation medium, **characterized in that** the at least one insert shelf (1) is an insert shelf (1) according to one of Claims 1 to 15.

17. Incubator (2) according to Claim 16, **characterized in that** the incubator (2) has a drive motor (18), preferably in the form of an electric motor, which is able to be coupled to a or the drive device (6) of the at least one insert shelf (1) and by way of which a or the movable set-up surface (5) of the at least one insert shelf (1), which, in the position of use, is inserted and/or slid into the incubator (2), is movable.

18. Incubator (2) according to either of Claims 16 and 17, **characterized in that** the drive motor (18) able to be coupled to the drive device (6) of the at least one insert shelf (1) is arranged on the incubator (2), in particular on a housing (38) of the incubator (2), and **in that** a drive torque of the drive motor (18) is able to be transmitted to the at least one insert shelf (1), in particular to the movable set-up surface (5), and/or to the swinging frame (11), preferably by means of a or the belt drive (19) of the insert shelf (1).

## Revendications

1. Tablette (1) pour recevoir au moins un récipient d'incubation, laquelle tablette (1) peut être engagée, en particulier insérée dans un incubateur (2) et peut être engagée et remplacée en position d'utilisation dans l'incubateur (2), et laquelle tablette (1) est pourvue d'un moyen (3) avec lequel un fluide d'incubation qui se trouve dans le récipient d'incubation placé sur la tablette (1) peut être mis en mouvement, **caractérisée en ce que**
a) le moyen de mise en mouvement du fluide d'incubation présente un entraînement d'aimant agitateur, la tablette (1) présentant un dispositif d'entraînement (6) avec lequel le fluide d'incubation se trouvant dans un récipient d'incubation placé sur la tablette (1) peut être mis en mouvement au moins indirectement et/ou
b) que la tablette (1) présente en tant qu'au moins un moyen (3) pour mettre en mouvement le fluide d'incubation une surface d'appui (5) mobile dans au moins une direction de déplacement pour recevoir au moins un récipient d'incubation et un châssis (4) fixe par rapport à la surface d'appui mobile (5), avec lequel la tablette (1) peut être fixée dans sa position d'utilisation insérée dans l'incubateur (2), la tablette (1) présentant un dispositif d'entraînement (6) avec lequel la surface d'appui (5) de la tablette (1) peut être mue au moins indirectement.

2. Tablette (1) selon la revendication 1, **caractérisée en ce que** l'au moins un moyen (3) peut être actionné électriquement et/ou, de préférence par rapport à un châssis fixe ou un châssis insérable (4) en position d'utilisation de la tablette (1), déplacé mécaniquement.

3. Tablette (1) selon la revendication 1 ou 2, **caractérisée en ce que** le châssis fixe (4) est un châssis insérable.

4. Tablette (1) selon une des revendications 2 à 3, **caractérisée en ce que** l'entraînement d'aimant agitateur (7) est configuré pour générer un champ magnétique circulant pour entraîner au moins un aimant agitateur pouvant être engagé dans un récipient d'incubation, en particulier l'au moins un entraînement d'aimant agitateur (7) pouvant être entraîné au moyen d'un ou du dispositif d'entraînement (6) de la tablette (1).

5. Tablette (1) selon une des revendications 2 à 4, **caractérisée en ce que** l'entraînement d'aimant agitateur (7) présente un disque de support monté de façon pivotante (8, 8a) avec au moins un, de préférence deux ou plusieurs aimants disposés sur le disque de support (8, 8a), le disque de support (8, 8a) pouvant de préférence être entraîné et mis en rotation mécaniquement au moyen du dispositif d'entraînement (6) de la tablette (1).

6. Tablette (1) selon une des revendications 1 à 5, **caractérisée en ce que** cette tablette (1) et/ou la surface d'appui (5) de la tablette (1) présente une multitude d'entraînements d'aimant agitateur (7) de préférence répartis régulièrement pour entraîner une multitude d'aimants agitateurs pouvant être engagés dans des récipients d'incubation.

7. Tablette (1) selon une des revendications 1 à 6, **caractérisée en ce que** la surface d'appui (5) est disposée sur un châssis oscillant (11) pouvant être mu au moyen d'un dispositif d'entraînement (6), en particulier sur sa face supérieure en position d'utilisation, le châssis oscillant (11) étant monté de façon mobile par rapport au châssis (4) de la tablette (1).

8. Tablette (1) selon une des revendications 1 à 7, **caractérisée en ce que** le châssis oscillant (11) présente une épaisseur ou une hauteur supérieure à celle du châssis (4) et/ou est disposé de sorte qu'il dépasse vers le haut le châssis (4) en position d'utilisation et/ou que la surface d'appui (5) mobile par rapport au châssis (4) est disposée dans un plan espacé, en particulier parallèle, par rapport à la face supérieure (17) du châssis (4).

9. Tablette (1) selon une des revendications 1 à 8, **caractérisée en ce que** cette tablette (1), en particulier le dispositif d'entraînement (6) de la tablette (1), présente un moteur d'entraînement (18), configuré de préférence comme un moteur électrique, qui est relié, de préférence par le dispositif d'entraînement (6), au moins indirectement à la surface d'appui (5) et/ou au châssis oscillant (11) et/ou à un ou à l'au moins un entraînement d'aimant agitateur (7) pour mettre la surface d'appui (5) et/ou le fluide d'incubation en mouvement.

10. Tablette (1) selon une des revendications 1 à 9, **caractérisée en ce que** cette tablette (1), en particulier le dispositif d'entraînement (6) de la tablette (1), présente une transmission à courroie (19) qui relie le châssis oscillant (11) et/ou la surface d'appui (5) de la tablette et/ou un ou l'au moins un entraînement d'aimant agitateur (7) au moins indirectement à un ou au moteur d'entraînement (18) et qu'au moyen de celle-ci, un couple d'entraînement du moteur d'entraînement (18) peut être transmis à la surface d'appui (5) mobile par rapport au châssis (4) de la tablette (1) et/ou au châssis oscillant (11) et/ou à un ou à l'au moins un entraînement d'aimant agitateur (7).

11. Tablette (1) selon une des revendications 1 à 10, **caractérisée en ce que** cette tablette (1), en particulier le dispositif d'entraînement (6) de la tablette (1), présente, pour mouvoir la surface d'appui et/ou le châssis oscillant (11) et/ou un ou l'au moins un entraînement d'aimant agitateur (7), une poulie de sortie (20) qui est reliée au moins indirectement à la surface d'appui (5) et/ou au châssis oscillant (11) et/ou à l'au moins un entraînement d'aimant agitateur (7) et est montée de façon pivotante par rapport à un ou au châssis fixe (4) de la tablette (1), et/ou que la poulie de sortie (20) est configurée de façon excentrique ou présente un tourillon excentré (21) disposé de façon excentrique sur la poulie de sortie (20) qui est relié au moins indirectement à la surface d'appui (5) et qu'au moyen de celui-ci, un mouvement de rotation de la poulie de sortie (20) peut être transformé en un mouvement excentrique du châssis oscillant (11) et de la surface d'appui (5).

12. Tablette (1) selon une des revendications 1 à 11, **caractérisée en ce que** le châssis (4) de cette tablette (1) comporte la surface d'appui (5) et/ou le châssis oscillant (11) de la tablette (1) de façon espacée au moins par endroits et/ou que cette tablette (1) présente une multitude d'orifices de convection (25) traversant la tablette (1), en particulier répartis régulièrement sur la tablette (1).

13. Tablette (1) selon une des revendications 1 à 12, **caractérisée en ce que** cette tablette (1) présente sur un côté (27) orienté vers une ouverture d'insertion (26) de l'incubateur (2) lorsque la tablette (1) est en position d'utilisation au moins une zone de préhension et/ou au moins une poignée de transport (28) et/ou au moins une poignée de serrage (29) avec laquelle la tablette (1) peut être fixée dans sa position d'utilisation engagée et/ou insérée dans l'incubateur (22).

14. Tablette (1) selon une des revendications 1 à 13, **caractérisée en ce que** cette tablette (1) est conçue comme un sous-plancher et peut être inséré dans un incubateur (2), en particulier dans des éléments de maintien d'un incubateur (2) configurés comme des rails de maintien (29), et/ou que cette tablette (1) présente, en particulier sur un côté (40) opposé à une ouverture d'insertion (26) de l'incubateur (2) lorsque la tablette (1) est en position d'utilisation, au moins un élément de centrage, en particulier au moins un tourillon de centrage (30) et/ou au moins un trou de centrage.

15. Tablette (1) selon une des revendications 1 à 13, **caractérisée en ce que** cette tablette (1) et/ou la surface d'appui (5) de cette tablette (1) présente au moins un élément de maintien (31), en particulier un tapis à picots et/ou une fixation de récipient d'incubation (32) pour assurer au moins un récipient d'incubation sur la surface d'appui (5) et/ou est dotée d'un revêtement antidérapant.

16. Incubateur (2) avec une chambre d'incubation chauffante (34) et avec au moins une tablette (1) remplaçable engagée et/ou insérée dans la chambre d'incubation (34) en position d'utilisation pour recevoir au moins un récipient d'incubation contenant un fluide d'incubation, **caractérisé en ce que** l'au moins une tablette (1) est une tablette selon une des revendications 1 à 15.

17. Incubateur (2) selon la revendication 16, **caractérisé en ce que** cet incubateur (2) présente un moteur d'entraînement (18) configuré de préférence comme un moteur électrique pouvant être accouplé avec un ou le dispositif d'entraînement (6) de l'au moins une tablette (1), avec lequel une ou la surface d'appui mobile (5) de l'au moins une tablette (1) engagée et/ou insérée en position d'utilisation dans l'incubateur (2) peut être mue.

18. Incubateur (2) selon une des revendications 16 à 17, **caractérisé en ce que** le moteur d'entraînement (18) pouvant être accouplé avec le dispositif d'entraînement (6) de l'au moins une tablette (1) est disposé sur cet incubateur (2), en particulier sur un boîtier de cet incubateur (2), et qu'un couple d'entraînement du moteur d'entraînement (18) peut être transmis à l'au moins une tablette (1), en particulier à la surface d'appui mobile (5) et/ou au châssis oscillant (11), de préférence au moyen d'une ou de la transmission à courroie (19) de la tablette (1).
